# FASCICULE DE BREVET EUROPEEN

(11) **EP 0 763 527 B1**
(45) Date de publication et mention de la délivrance du brevet: **26.04.2000**
(21) Numéro de dépôt: 96401946.7
(22) Date de dépôt: 12.09.1996
(51) Int. Cl.: C07C 323/16, C07C 59/68, C07D 311/72, A61K 31/19, A61K 31/35

(54) **Nouveaux acides et esters 2,2-diméthyl-oméga-phénoxy alcanoiques substitués, leur procédé de préparation et les compositions pharmaceutiques qui les contiennent**
Substituierte 2,2-Dimethyl-omega-Phenoxysäure und -ester, Verfahren zu ihrer Herstellung und diese enthaltende pharmazeutische Zusammensetzungen
Substituted 2,2-dimethyl-omega-phenoxy alkanoic acids and esters, process for their preparation and pharmaceutical compositions containing them

(30) Priorité: 14.09.1995 FR 9510731
(43) Date de publication de la demande: 19.03.1997
(73) Titulaire: ADIR ET COMPAGNIE, 92415 Courbevoie Cédex (FR)
(72) Inventeur: Regnier, Gilbert, 92290 Chatenay Malabry (FR); Guillonneau, Claude, 92140 Clamart (FR); Vilaine, Jean-Paul, 92290 Chatenay Malabry (FR); Mahlberg, Florence, 92210 Saint Cloud (FR); Breugnot, Christine, 75015 Paris (FR)
(74) Mandataire: Reverbori, Marcelle

(56) Documents cités:
- US-A- 4 351 950
- DATABASE WPI Section Ch, Week 8742 Derwent Publications Ltd., London, GB; Class B05, AN 87-295479 XP002003703 & JP 62 207 236 A (SANKYO) , 11 Septembre 1987
- JOURNAL OF MEDICINAL CHEMISTRY, vol. 32, no. 2, Février 1989, WASHINGTON, DC, US, pages 421-428, XP002003773 T. YOSHIOKA, ET AL.: "Studies on hindered phenols and analogues. 1. Hypolipidaemic and hypoglycaemic agents with ability to inhibit lipid peroxidaton"
- JOURNAL OF MEDICINAL CHEMISTRY, vol. 31, no. 6, Juin 1988, WASHINGTON, DC, US, pages 1205-1209, XP002003702 S. MORISHITA, ET AL.: "Synthesis and hypolipidaemic activity of 2-substituted isobutyric acid derivatives"

## Description

La présente invention a pour objet de nouveaux acides et esters 2,2-diméthyl-ω-phénoxy alcanoïques substitués, leur procédé de préparation et les compositions pharmaceutiques les contenant.

Elle concerne plus particulièrement les acides et esters 2,2-diméthyl ω-phénoxy alcanoïques substitués de formule I: 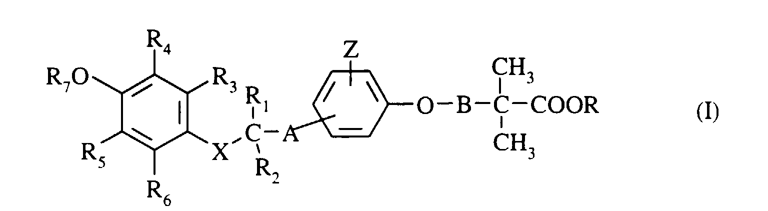 dans laquelle :
- **X**: représente un atome d'oxygène, un atome de soufre ou une liaison simple ;
- **A**: représente une liaison simple ou un radical hydrocarboné contenant de 1 à 9 atomes de carbone en chaîne droite ou ramifiée renfermant éventuellement une double liaison ou un atome d'oxygène ;
- **B**: représente un radical hydrocarboné contenant de 1 à 9 atomes de carbone en chaîne droite ou ramifiée;
- **R**: représente un atome d'hydrogène ou un radical alkyle de 1 à 6 atomes de carbone en chaîne droite ou ramifiée, éventuellement substitué par un ou deux radicaux hydroxy ;
- **R**_{**1**} **et R**_{**3**}: représentent:
- chacun simultanément un atome d'hydrogène, ou
- forment ensemble un pont (CH₂)ₙ dans lequel n prend les valeurs 1 ou 2 sauf dans le cas où X représente une liaison simple, ou
- R₁ représente
   - un radical méthyle, ou
   - une liaison simple formant une double liaison avec le groupe A lorsque celui--ci est un radical hydrocarboné et,

. dans chacun de ces cas, simultanément R₃ représente un atome d'hydrogène ;
- **R**_{**2**} **et R**_{**6**}: identiques ou différents, représentent chacun un atome d'hydrogène ou un radical méthyle ;
- **R**_{**4**} **et R**_{**5**}: identiques ou différents, représentent chacun un radical alkyle de 1 à 6 atomes de carbone en chaîne droite ou ramifiée;
- **R**_{**7**}: représente un atome d'hydrogène ou un groupement protecteur labile tel que par exemple, un radical CH₃CO-, C₂H₅OCH₂- ou benzyle ; et
- **Z**: représente un atome d'hydrogène ou d'halogène ou un radical alkyle ou alkoxy contenant chacun de 1 à 5 atomes de carbone en chaîne droite ou ramifiée.

Certains des composés de formule I comportent un ou plusieurs atomes chiraux et peuvent ainsi exister sous forme d'énantiomères ou de diastéréoisomères qui font également partie de la présente invention. D'autre part, les composés de formule I qui renferment une double liaison peuvent exister sous forme E ou Z qui font chacune partie de la présente invention. De même, les composés de formule I dans laquelle R représente un atome d'hydrogène peuvent être transformés en sels d'addition avec des bases pharmaceutiquement acceptables, sels qui sont, à ce titre, inclus dans la présente invention.

L'état antérieur de la technique le plus proche de la présente invention est notamment illustré par la demande de brevet JP 62207236, ainsi que par l'article *J.Med. Chem.* 1989, 32(2), 421-428. Ces deux documents décrivent des dérivés d'acide phénoxyalcanoïque utiles pour le traitement de l'hyperlipidémie. Ces dérivés se distinguent largement des composés revendiqués par la Demanderesse dans la présente invention, notamment par l'absence au sein de la structure d'un second groupement benzénique.

Le document *J.Med. Chem,* 1988, 31(6), 1205-1209 décrit quant à lui des dérivés d'acide isobutyrique de formule générale (α) : 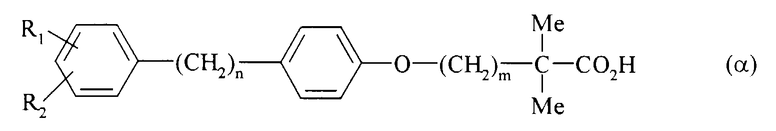 dans laquelle m et n sont des entiers compris respectivement entre 3 et 10, et 1 et 6, et R₁ et R₂, identiques, représentent un atome d'hydrogène ou un groupement méthyle. Ces composés sont utiles pour le traitement de l'hyperlipidémie.

Enfin, le brevet US 4,752,616 a pour objet, entre autres, des acides et esters thioalkylphényles alcanoïques qui constituent des agents anti-thrombotiques, antiasthmatiques et vasodilatateurs.

Les composés de la présente invention diffèrent des composés antérieurement connus ci-dessus définis à la fois par leur structure chimique et par leur activité pharmacologique et thérapeutique.

La présente invention a également pour objet le procédé de préparation des composés de formule I caractérisé en ce que l'on fait réagir :
**a)**
   . soit un composé de formule IIa : 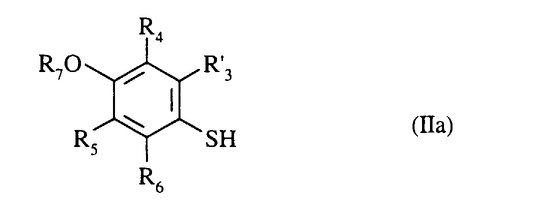 dans laquelle :
      R₄, R₅, R₆ et R₇ ont les significations précédemment définies et
      R'₃ représente un atome d'hydrogène,
   . avec un composé de formule IIIa : 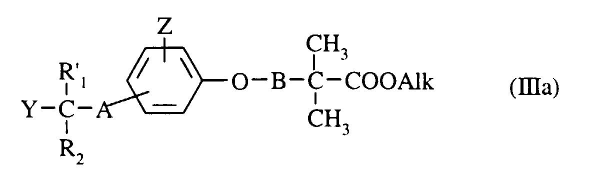 dans laquelle :
      A, B, R₂ et Z ont les significations précédemment définies,
      R'₁ représente un atome d'hydrogène ou un radical méthyle ;
      Alk représente un radical alkyle de 1 à 6 atomes de carbone en chaîne droite ou ramifiée, et
      Y représente un atome de chlore ou de brome ;
   . pour obtenir un composé de formule Ia₁ : 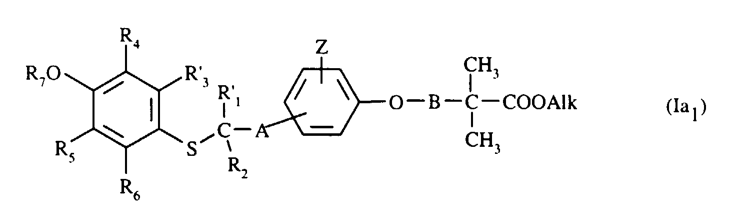 dans laquelle R'₁, R₂, R'₃, R₄, R₅, R₆, R₇, A, Z, B et Alk ont les significations précédemment définies,
   . lequel composé Ia₁ est, selon la nature de R₇, transformé par des méthodes séquentielles de saponification, hydrolyse ou hydrogénolyse, en un composé de formule Ia₂ : 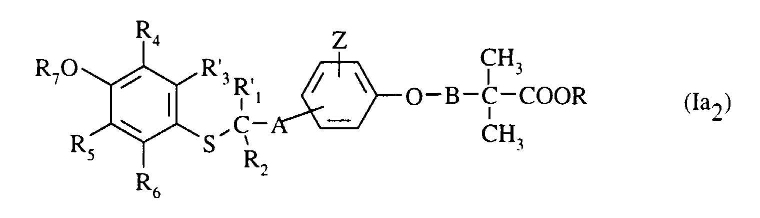 dans laquelle R'₁, R₂, R'₃, R₄, R₅, R₆, R₇, A, Z, B et R ont les significations précédemment définies ;
**b)**
   . soit un composé de formule IIb : 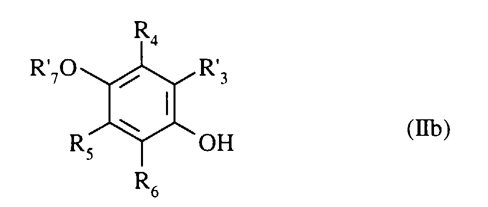 dans laquelle :
      R'₃, R₄, R₅ et R₆ ont les significations précédemment définies, et
      R'₇ représente un groupement protecteur labile tel que CH₃-CO-, C₂H₅-O-CH₂ ou benzyle ;
   . avec un composé de formule IIIb : 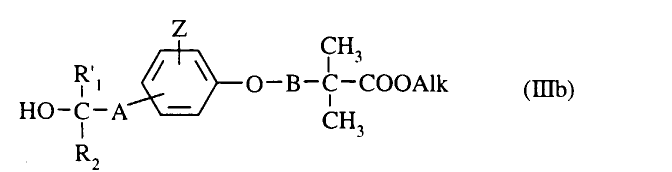 dans laquelle R'₁, R₂, A, Z, B et Alk ont les significations précédemment définies,
   . pour obtenir un composé de formule Ib₁ : 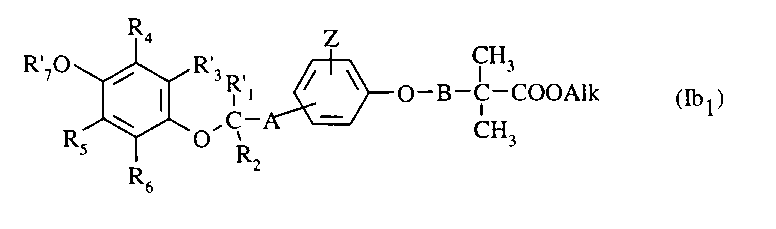 dans laquelle R'₁, R₂, R'₃, R₄, R₅, R₆, R'₇, A, Z, B et Alk ont les significations précédemment définies,
   . lequel composé de formule Ib₁ est, selon la nature de R'₇, transformé par des méthodes séquentielles de saponification, hydrolyse ou hydrogénolyse, en un composé de formule Ib₂: 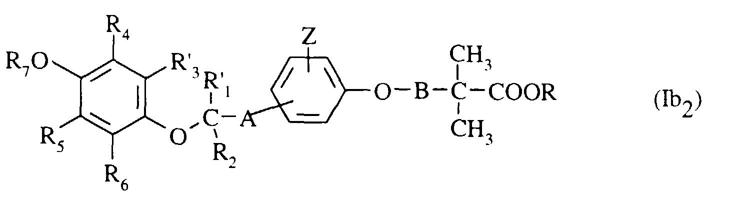 dans laquelle R'₁, R₂, R'₃, R₄, R₅, R₆, R₇, A, Z, B et R ont les significations précédemment définies ;
c)
   . soit un composé de formule IIc : 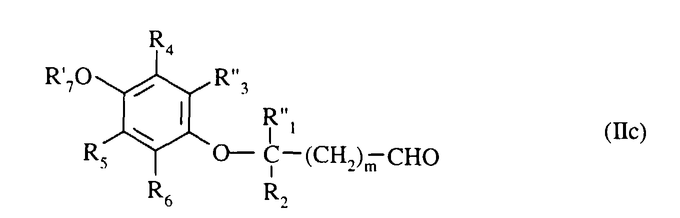 dans laquelle :
      R₂, R₄, R₅, R₆ et R'₇ ont les significations précédemment définies,
      R"₁ et R"₃ représentent ensemble un pont (CH₂)ₙ dans lequel n prend la signification précédemment définie, et
      m représente zéro ou un nombre entier de 1 à 5 inclus ;
   . avec un composé de formule IIIc : 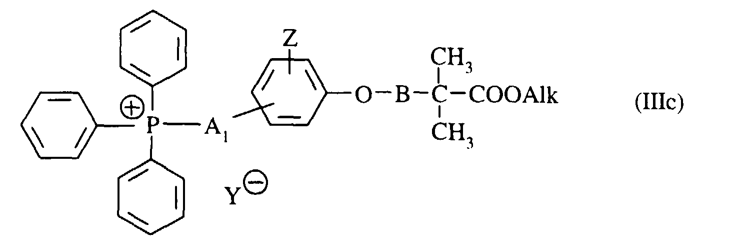 dans laquelle :
      Alk, B, Z et Y ont les significations précédemment défines, et
      A₁ représente un radical hydrocarboné contenant de 1 à 3 atomes de carbone en chaîne droite ou ramifiée ;
   . pour obtenir un composé de formule Ic₁ : 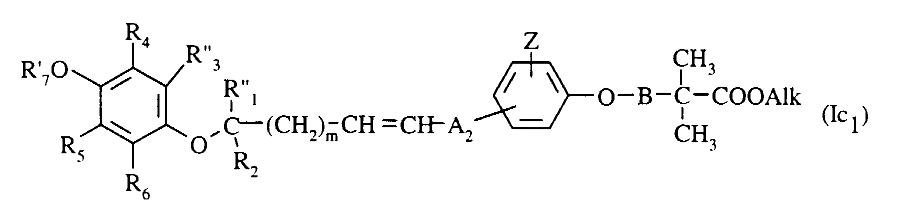 dans laquelle :
      R"₁, R₂, R"₃, R₄, R₅, R₆, R'₇, m, Z, B et Alk ont les significations précédemment définies, et
      A₂ représente une liaison simple ou un radical hydrocarboné contenant 1 ou 2 atomes de carbone en chaîne droite ou ramifiée ;
   . lequel composé de formule Ic₁ est réduit pour obtenir le composé de formule Ic₂ : 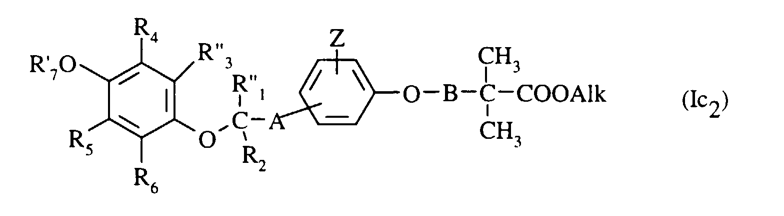 dans laquelle R"₁, R₂, R"₃, R₄, R₅, R₆, R'₇, A, Z, B et Alk ont les significations précédemment définies ;
   . lequel composé de formule Ic₂ est, selon la nature de R'₇, transformé, par des méthodes séquentielles de saponification, hydrolyse ou hydrogénolyse en un composé de formule Ic₃ : 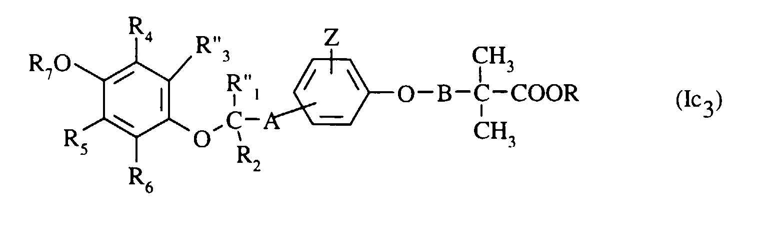 dans laquelle R"₁, R₂, R"₃, R₄, R₅, R₆, R₇, A, Z, B et R ont les significations précédemment définies.

L'ensemble des composés de formule Ia₁, Ia₂, Ib₁, Ib₂, Ic₁, Ic₂ et Ic₃ forme l'ensemble des composés de formule I.

Il est particulièrement avantageux de faire réagir les composés de formules respectives IIa et IIIa en présence d'un accepteur de l'hydracide formé au cours de la réaction dans un solvant tel que par exemple l'acétone, l'acétonitrile ou le diméthylformamide à une température comprise entre 50 et 120 °C.

Comme accepteur, on peut utiliser, par exemple, un carbonate alcalin en présence d'un iodure alcalin, la diméthylaminopyridine ou la triéthylamine.

La réaction des composés de formules respectives IIb et IIIb s'effectue selon la technique de O. Mitsunobu, Synthesis (1981), 1-28, en utilisant comme réactifs l'azodicarboxylate d'éthyle et la triphénylphosphine et en opérant dans un solvant aprotique tel que par exemple le tétrahydrofurane ou l'éther à une température comprise entre 20 et 25 °C.

La réaction des composés de formules respectives IIc et IIIc s'effectue avantageusement selon la technique de Wittig G., Ann. (1953), 580, 44 et Bruce et coll., Chem. Rev. (1989), 863-927, en utilisant le butyl lithium comme réactif et en opérant en milieu tétrahydrofurane, à une température comprise entre 20 et 25 °C.

Une variante de cette technique, qui conduit à des rendements supérieurs, s'effectue selon Buddrus, Chem. Ber. (1974), 107, 2050-61. On opère dans ce cas en présence de 1,2-époxybutane en excès qui sert à la fois de réactif et de solvant, à la température de reflux (63 °C).

L'hydrogénation catalytique du composé Ic₁ s'effectue au moyen de charbon palladié sous une pression de 5.10⁵ Pa, en opérant dans l'éthanol à une température comprise entre 20 et 25 °C.

Les matières premières de formules IIa et IIb sont des produits commerciaux déjà décrits dans la littérature.

Les matières premières de formule IIc sont aussi décrites dans la littérature et préparées selon N. Cohen et coll., J. Am. Chem. Soc. 101, 6710-15 (1979) ou selon Takeda, E.P. 345 593.

Les matières premières de formule IIIb sont obtenues selon le procédé qui consiste à déprotéger, en milieu acide par exemple dans l'éthanol chlorhydrique, un composé de formule IV : 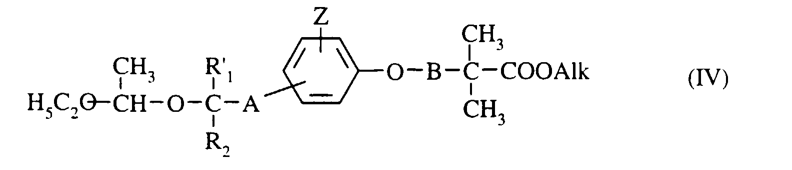 dans laquelle R'₁, R₂, A, Z, B et Alk ont les significations précédemment définies.

Ce composé IV est lui-même obtenu en faisant réagir un composé de formule V : 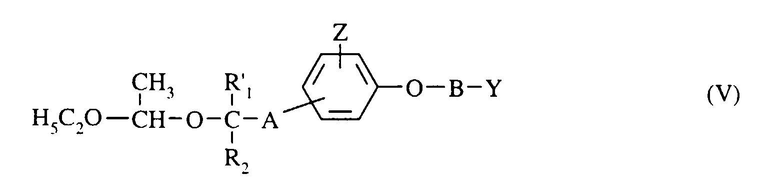 dans laquelle R'₁, R₂, A, Z, B et Y ont les significations précédemment définies,
avec un isobutyrate d'alkyle, comme par exemple l'isobutyrate d'éthyle, en présence d'une base forte, comme par exemple le diisopropylamidure de lithium, dans un solvant aprotique, comme par exemple le tétrahydrofurane.

Ce composé de formule V est obtenu en faisant réagir un composé de formule VI : 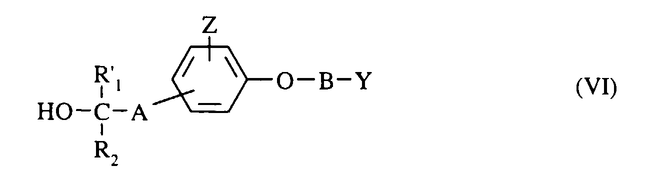 dans laquelle R'₁, R₂, A, Z, B et Y ont les significations précédemment définies,
avec l'éthylvinyl éther dans un solvant aprotique, comme par exemple le dichlorométhane, en présence d'un hydracide ou d'un acide fort, comme par exemple l'acide trichloroacétique.

Le composé de formule VI est lui-même obtenu en faisant réagir un composé de formule VII : 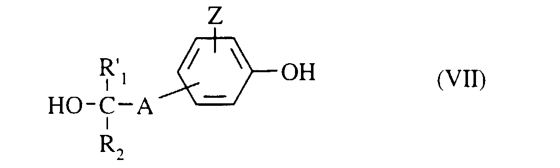 dans laquelle R'₁, R₂, A et Z ont les significations précédemment définies,
avec un ω-dichloro- ou un ω-dibromo-alcane, dans un solvant aprotique polaire tel que par exemple la méthyl isobutyl cétone, en présence d'un carbonate alcalin.

Les matières premières de formule IIIa sont obtenues en faisant réagir les composés de formule IIIb avec la triphényl phosphine en présence de CCl₄ ou de brome dans l'acétonitrile selon la méthode de J. Hooz et coll., Can. J. Chem. 46, 86-7 (1968) ou de J. Schaefer et coll., Org. Synth. collect. vol V, 249.

Les matières premières de formule IIIc sont préparées sous forme non cristallisée, amorphe, selon le procédé classique qui consiste à faire réagir un composé de formule IIIa telle que précédemment définie, avec la triphénylphosphine, à reflux, dans un solvant aprotique polaire comme par exemple l'acétonitrile.

Les composés de formule I ainsi obtenus peuvent être purifiés par chromatoflash sur silice (35-70 µ) en utilisant comme éluant l'acétate d'éthyle ou le mélange CH₂Cl₂/CH₃OH, ou par formation de sels et cristallisation de ceux-ci.

Certains composés de formule I donnent des sels avec des bases physiologiquement tolérables - sels qui sont, à ce titre, inclus dans la présente invention.

Les composés de la présente invention possèdent des activités pharmacologiques et thérapeutiques intéressantes, notamment une activité protectrice de l'oxydation des LDL (lipoprotéines de basse densité) humaines et une activité hypolipémiante qui permettent leur utilisation comme médicament en particulier dans le traitement :
- des hypercholestérolémies,
- des hypertriglycéridémies,
- des dyslipémies et du diabète, pour prévenir les complications notamment vasculaires,
- de l'athérosclérose avec ses différentes localisations vasculaires : périphériques, coronaires ou cérébrales,
- mais aussi dans les pathologies dans lesquelles une peroxydation lipidique membranaire joue un rôle initiateur et/ou aggravant telles les cardiopathies ischémiques, la reperfusion d'organes, y compris transplantés, les pathologies ischémiques traumatiques ou dégénératives du système nerveux central ou périphérique, les maladies inflammatoires aigües ou chroniques et les maladies auto-immunes.

La présente invention a également pour objet les compositions pharmaceutiques contenant comme principe actif un composé de formule I ou un de ses sels physiologiquement tolérable mélangé ou associé à un excipient pharmaceutique approprié comme, par exemple, le glucose, le lactose, l'amidon, le talc, l'éthylcellulose, le stéarate de magnésium ou le beurre de cacao.

Ces compositions pharmaceutiques se présentent généralement sous forme dosée et peuvent contenir de 100 à 500 mg de principe actif.

Elles peuvent revêtir, par exemple, la forme de comprimés, dragées, gélules, suppositoires, solutions injectables ou buvables, et être selon les cas, administrées par voie orale, rectale ou parentérale à la dose de 100 à 1 500 mg en 1 à 3 prises quotidiennes.

Les exemples suivants illustrent la présente invention, les points de fusion étant déterminés à la platine chauffante de Kofler (K) ou au tube capillaire (cap).

### Exemple 1

Acide 2,2-diméthyl-5-{4-[2-(3,5-ditert-butyl-4-hydroxyphénylthio)éthyl]phénoxy} pentanoïque, et son sel de tert-butylamine.

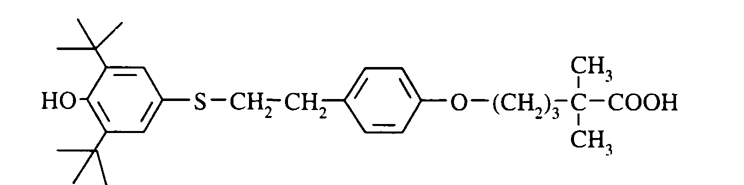

On porte au reflux 6,9 g (0,02 mole) de 2,2-diméthyl-5-[4-(2-bromoéthyl)phénoxy]pentanoate d'éthyle, 5,3 g (0,022 mole) de 3,5-ditert-butyl-4-hydroxy phénylthiol, 3 g de carbonate dipotassique, 0,3 g d'iodure de potassium et 200 ml d'acétone, et maintient le reflux pendant 20 heures. On filtre, concentre à sec, reprend le résidu dans le dichlorométhane, lave à l'eau et sèche sur sulfate de sodium.

Après concentration à sec, le résidu est chromatographié sur silice en éluant avec un mélange de dichlorométhane et de cyclohexane (50-50). On obtient 9,35 g de l'ester attendu sous forme de gomme (Rendement : 91 %). On porte au reflux 5,9 g (0,0115 moles) de 2,2-diméthyl-5-{4-[2-(3,5-ditert-butyl4-hydroxy phényl thio)éthyl]phénoxy}pentanoate d'éthyle, 16,1 ml de soude N et 100 ml d'éthanol et maintient le reflux pendant 24 heures. Après refroidissement, le mélange réactionnel est acidifié par l'acide chlorhydrique N, puis concentré à sec. Le résidu est repris à l'éther, lavé à l'eau, séché sur sulfate de sodium et concentré à sec. Le résidu est chromatographié sur 180 g de silice en éluant par un mélange (95-5) de dichlorométhane et d'acétone.
L'acide attendu est obtenu sous forme de gomme que l'on dissout dans l'éther et additionne d'un excès de tert-butylamine. On observe une cristallisation. Après filtration, essorage et séchage à 40 °C, sous une pression de 133 Pa, on obtient 4,6 g de sel de tert-butylamine de l'acide 2,2-diméthyl-5-{4-[2-(3,5-ditert-butyl-4-hydroxyphénylthio)éthyl]phénoxy} pentanoïque, PF(cap) : 118-121 °C (Rendement : 72 %).

### Exemples 2-8

En opérant comme décrit dans l'exemple 1 ont été préparés les composés objet des exemples suivants :
2) L'acide 2,2-diméthyl-5-{4-[2-(3,5-ditert-butyl-4-hydroxyphénylthio)éthyl]phénoxy} pentanoïque et son sel de tert-butylamine, PF(K) : 110 °C (éther).
3) L'acide 2,2-diméthyl-5-{4-[3-(3,5-ditert-butyl-4-hydroxyphénylthio)propyl]phénoxy} pentanoïque et son sel de tert-butylamine, PF(cap) : 132-138 °C (éther-pentane).
4) L'acide 2,2-diméthyl-5-{4-[3-(4-hydroxy-2,3,5-triméthylphénylthio)propyl]phénoxy} pentanoïque, PF (cap) : 68-70 °C (dichlorométhane).
5) L'acide 2,2-diméthyl-5-{4-[3-(3,5-ditert-butyl-4-hydroxyphénylthio)propyloxy]phénoxy} pentanoïque et son sel de tert-butylamine PF. (cap) : 135-138 °C (éther).
6) L'acide 2,2-diméthyl-5-{4-[3-(4-hydroxy-2,3,5-triméthylphénylthio)propyloxy]phénoxy} pentanoïque et son sel de tert-butylamine, PF (cap) : 122-126 °C (éther).
7) L'acide 2,2-diméthyl-5-{3-[2-(4-hydroxy-2,3,5-triméthylphénylthio)éthyl]phénoxy} pentanoïque, PF (cap) : 72-76 °C (dichlorométhane).
8) L'acide 2,2-diméthyl-5-{3-[2-(3,5-ditert-butyl-4-hydroxyphénylthio)éthyl]phénoxy} pentanoïque, PF (cap) : 102-103 °C (dichlorométhane-acétone).

### Exemple 9

Acide 2,2-diméthyl-5-{4-[2-(4-hydroxy-2,3,5-triméthylphénoxy)éthyl]phénoxy}pentanoïque, et son sel de tert-butylamine.

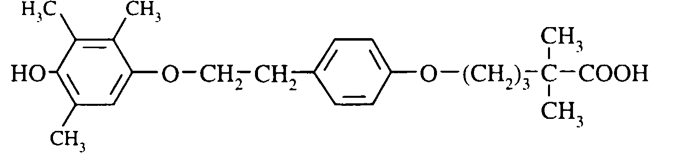

On coule goutte à goutte 15,67 g d'azodicarboxylate d'éthyle dans un mélange contenant 11,6 g (0,06 mole) de 4-acétoxy-2,3,5-triméthylphénol fondant (K) à 108 °C, 23,6 g de triphénylphosphine, 500 ml de tétrahydrofurane et 19,2 g (0,065 mole) de 2,2-diméthyl-5-[4-(2-hydroxyéthyl)phénoxy]pentanoate d'éthyle. On agite le tout une nuit à température ambiante puis on concentre à sec. On ajoute du cyclohexane, triture et filtre l'insoluble. Le filtrat est concentré à sec et chromatographié sur 1,16 kg de silice en éluant avec du dichlorométhane. On obtient 10,9 g de l'ester attendu, sous forme d'huile épaisse (Rendement: 39 %).
On porte au reflux pendant 3 heures, 10,9 g de 2,2-diméthyl-5-{4-[2-(4-acétoxy-2,3,5-triméthylphénoxy)éthyl]phénoxy}pentanoate d'éthyle avec 500 ml d'éthanol et 80 ml de soude N, sous atmosphère d'azote. Après refroidissement, on acidifie par 100 ml d'acide chlorhydrique N puis concentre à sec la solution . Le résidu est repris dans l'éther. La solution obtenue est lavée à l'eau, séchée sur sulfate de sodium et concentrée à sec. Le résidu est chromatographié sur 340 g de silice en éluant avec un mélange dichlorométhane-acétone (90-10). On obtient 4,4 g de l'acide souhaité, sous forme de gomme que l'on dissout dans 50 ml d'éther éthylique.
On ajoute à cette solution, 1,3 ml de tert-butylamine. On observe une cristallisation. Le sel ainsi formé est filtré, essoré, rincé à l'éther puis séché à 50 °C sous une pression de 133 Pa. On obtient 2,65 g de sel de tert-butylamine de l'acide 2,2-diméthyl-5-{4-[2-(4-hydroxy-2,3,5-triméthylphénoxy)éthyl]phénoxy}pentanoïque, PF (K) : 126 °C (Rendement : 24,5 %).

### Exemples 10-16

En opérant comme décrit dans l'exemple 9 ont été préparés les composés objet des exemples suivants :
10) L'acide 2,2-diméthyl-5-{4-[3-(3,5-di-tert-butyl-4-hydroxyphénoxy)propyl]phénoxy} pentanoïque, PF (cap) : 118-121 °C (dichlorométhane).
11) L'acide 2,2-diméthyl-5-{4-[3-(4-hydroxy-2,3,5-triméthylphénoxy)propyloxy]phénoxy} pentanoïque, PF : 80-85 °C (dichlorométhane-acétone).
12) L'acide 2,2-diméthyl-5-{4-[3-(3,5-ditert-butyl-4-hydroxyphénoxy)propyloxy]phénoxy} pentanoïque et son sel de tert-butylamine PF (cap) : 146-149 °C (éther).
13) L'acide 2,2-diméthyl-5-{3-[2-(3,5-ditert-butyl-4-hydroxyphénoxy)éthyl]phénoxy} pentanoïque et son sel de tert-butylamine PF (cap) : 121-125 °C (éther).
14) L'acide 2,2-diméthyl-5-{3-[2-(4-hydroxy-2,3,5-triméthylphénoxy)éthyl]phénoxy} pentanoïque et son sel de tert-butylamine PF (cap) : 118-120 °C (éther-pentane).
15) L'acide 2,2-diméthyl-5-{4-[3-(4-hydroxy-2,3,5-triméthylphénoxy)propyl]phénoxy} pentanoïque et son sel de tert-butylamine PF (cap) : 126-130 °C (éther).
16) L'acide 2,2-diméthyl-5-{4-[2-(4-hydroxy-3,5-ditert-butylphénoxy)éthyl]phénoxy} pentanoïque PF (K) : 114 °C (éthanol).

### Exemple 17

L'acide R,S-2,2-diméthyl-5-{4-[4-(6-hydroxy-2,5,7,8-tétraméthyl-3,4-dihydrobenzopyran-2-yl)but-3-ényloxy]phénoxy} pentanoïque.

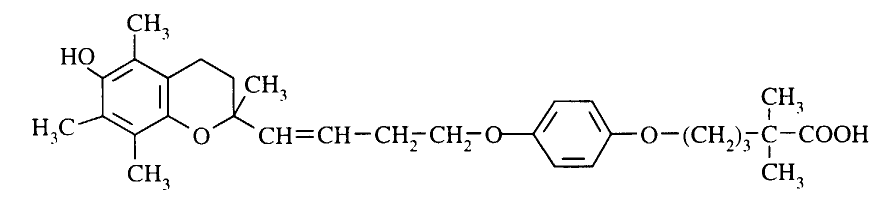

On porte au reflux pendant 72 heures un mélange contenant 27,3 g (0,042 mole) de bromure de 3-[4-(4,4-diméthyl-4-éthoxycarbonyl butoxy)phénoxy]propyl triphényl phosphonium, 12,2 g (0,042 mole) de 6-éthoxyméthoxy-2-formyl-2,5,7,8-tétraméthyl-3,4-dihydrobenzopyrane et 1,2 l de 1,2-époxybutane. On concentre à sec et chromatographie le résidu sur 1,1 kg de silice en éluant avec un mélange de dichlorométhane et de cyclohexane (50-50). On obtient 16,6 g de R,S-2,2-diméthyl-5-{4-[4-(6-éthoxyméthoxy-2,5,7,8-tétraméthyl-3,4-dihydrobenzopyran-2-yl)but-3-ényloxy]phénoxy}pentanoate d'éthyle, sous forme de mélange E et Z gommeux (Rendement : 67 %).
Le 6-éthoxyméthoxy-2-formyl-2,5,7,8-tétraméthylchromane de départ a été préparé par réduction, à l'hydrure de diisobutyl aluminium, de l'ester méthylique correspondant (huile n_{D}^{20 °C} = 1,5207) lui-même préparé à partir de l'ester méthylique du Trolox et du chlorométhyl éthyl éther dans le diméthylformamide en présence d'hydrure de sodium.
Le bromure de 3-[4-(4,4-diméthyl-4-éthoxycarbonyl butoxy)phénoxy]propyltriphényl phosphonium de départ a été préparé par réaction de la triphényl phosphine, dans l'acétonitrile à reflux, avec le2,2-diméthyl-5-[4-(3-bromopropyloxy)phénoxy]pentanoate d'éthyle, lui-même obtenu à partir de 5-[4-(3-hydroxypropyloxy)phénoxy]pentanoate d'éthyle et de brome dans l'acétonitrile en présence de triphénylphosphine.
Le R,S-2,2-diméthyl-5-{4-[4-(6-éthoxyméthoxy-2,5,7,8-tétraméthyl-3,4-dihydrobenzopyran-2-yl)but-3-ényloxy]phénoxy}pentanoate d'éthyle précédemment obtenu, est saponifié dans un mélange d'éthanol et de soude N au reflux et déprotégé dans du dioxane chlorhydrique 4N. On obtient 4,8 g de l'acide attendu, PF (cap) : 70-75 °C (éther de pétrole) (Rendement : 35 %).

### Exemple 18

L'acide R,S-2,2-diméthyl-5-{4-[4-(6-hydroxy-2,,5,7,8-tétraméthyl-3,4-dihydrobenzopyran-2-yl)butyloxy]phénoxy} pentanoïque. 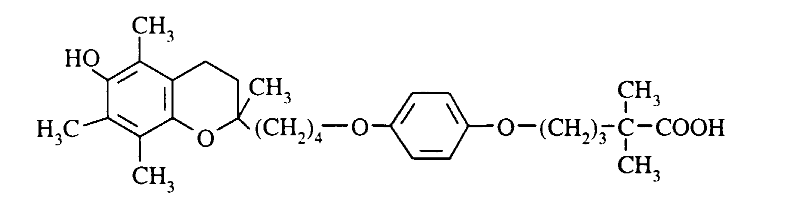 On hydrogène 6,8 g (0,0137 mole) d'acide R,S-2,2-diméthyl-5-{4-[4-(6-hydroxy-2,5,7,8-tétraméthyl-3,4-dihydrobenzopyran-2-yl)but-3-ényloxy]phénoxy}pentanoïque (préparé selon l'exemple 17) en solution dans 200 ml d'éthanol en présence de 1 g de palladium sur charbon à 5 % dans un appareil de Parr sous une pression de 5.10⁵ Pa à température ambiante. On filtre et concentre à sec puis chromatographie sur 340 g de silice en éluant avec un mélange toluène-tétrahydrofurane (90-10). On obtient 2,65 g de l'acide attendu, PF (cap) : 90-94 °C (Rendement : 39 %).

### Exemples 19-20

En opérant comme décrit dans l'exemple 18, ont été préparés les composés objet des exemples suivants :
19) L'acide R, S-2,2-diméthyl-5-{3-[3-(6-hydroxy-2,5,7,8-tétraméthyl-3,4-dihydrobenzopyran - 2-yl)propyl ]phénoxy}pentanoique et son sel de sodium sous forme de lyophilisat.
Le R,S-2,2-diméthyl-5-{3-[3-(6-éthoxy-2,5,7,8-tétraméthyl-3,4-dihydrobenzopyran-2-yl) propyl]phénoxy}pentanoate d'éthyle intermédiaire a été préparé selon la méthode décrite dans l'exemple 17.
20) L'acide 2,2-diméthyl-5-{3-[3-(4-hydroxy-3,5-ditert-butylphényl)propyl]phénoxy} pentanoïque et son sel de tert-butylamine PF (cap) : 97-100 °C (pentane).
Le 2,2-diméthyl-5-{3-[3-(4-éthoxyméthoxy-3,5-ditert-butylphényl)propyl]phénoxy} pentanoate d'éthyle intermédiaire a été préparé selon la méthode décrite dans l'exemple 17.

### Exemple 21

### Etude pharmacologique

### 1. ACTIVITE PROTECTRICE DE L'OXYDATION DES LDL

L'effet anti-oxydant des composés de la présente invention a été démontré *in vitro,* sur la peroxydation des lipoprotéines de basse densité (LDL) humaines induite par le sulfate de cuivre. La relation effet-dose de ces composés a été comparée à celle du probucol et d'un analogue soluble de la vitamine E, le trolox.

### A. METHODES

L'activité antioxydante des composés a été mesurée selon la méthode de Wallin et al. (Analytical Biochemistry, 1993, 208, 10-15).

### a) Réaction d'oxydation des LDL

Les LDL humaines sont incubées pendant 3 heures à 37°C en présence d'un agent oxydant, le sulfate de cuivre à 5 µM, et du composé à tester à des concentrations allant de 10⁻⁸ M à 2,5 x 10⁻⁶ M. Dans chaque série de tests, un contrôle non oxydé (LDL seules) et un contrôle oxydé (LDL plus sulfate de cuivre) sont également inclus.

### b) Dosage des TBARS

Les produits de la réaction d'oxydation des LDL sont quantifiés par le dosage colorimétrique des "thiobarbituric acid reactive substances" (TBARS).

### B. RESULTATS

L'activité protectrice des composés testés est exprimée par la valeur de l'IC₅₀, qui correspond à la concentration de produit nécessaire pour inhiber de 50% l'oxydation des LDL observée en absence de produit. La valeur de l'IC₅₀ a été calculée à partir de la courbe dose-réponse.
Les IC₅₀ obtenues pour chacun des composés testés sont présentées dans le Tableau A. Tous les composés testés sont plus actifs que les composés de référence et les composés des exemples 1 à 19 ont une IC₅₀ variant entre 1,7 x 10⁻⁷M et 6,8 x 10⁻⁸M. Ces résultats indiquent une activité protectrice contre l'oxydation des LDL de 10 à 70 fois supérieure à celle exercée par les composés de référence, probucol et trolox.

**Tableau A**

| **Protection contre l'oxydation des LDL** | |
|---|---|
| **Composés** | **IC 50 (M)** |
| Exemple 1 | 4,2 x 10⁻⁷ |
| Exemple 2 | 2,1 x 10⁻⁷ |
| Exemple 3 | 5,3 x 10⁻⁷ |
| Exemple 4 | 4,6 x 10⁻⁷ |
| Exemple 5 | 5,5 x 10⁻⁷ |
| Exemple 6 | 1,7 x 10⁻⁷ |
| Exemple 7 | 6,8 x 10⁻⁸ |
| Exemple 8 | 3,8 x 10⁻⁷ |
| Exemple 9 | 2,0 x 10⁻⁷ |
| Exemple 10 | 2,4 x 10⁻⁷ |
| Exemple 11 | 1,9 x 10⁻⁷ |
| Exemple 12 | 6,4 x 10⁻⁷ |
| Exemple 13 | 3 x 10⁻⁷ |
| Exemple 14 | 8,8 x 10⁻⁸ |
| Exemple 15 | 9,7 x 10⁻⁸ |
| Exemple 16 | 7,7 x 10⁻⁷ |
| Exemple 17 | 9.6 x 10⁻⁷ |
| Exemple 18 | 7.2 x 10⁻⁷ |
| Exemple 19 | 6,8 x 10⁻⁷ |
| Exemple 20 | 1,1 x 10⁻⁶ |
| Probucol | 5,8 x 10⁻⁶ |
| Trolox | 1,6 x 10⁻⁶ |

### 2. ACTIVITE HYPOLIPEMIANTE

L'activité hypolipémiante de certains composés représentatifs de l'invention a été évaluée *in vivo* dans un modèle d'hyperlipémie combinée induite par le régime chez le hamster.

### A. METHODES

Les hamsters Golden Syrian sont soumis à un régime riche en lipides (régime standard + 0.5% de cholestérol + 10% d'huile de coco) pendant 3 semaines avant le début des traitements. Ce régime induit une hypercholestérolémie et une hypertriglycéridémie.

Les produits sont préparés dans la gomme adragante et sont administrés *per os* par gavage gastrique pendant 1 semaine à la dose de 200 mg/kg/jour. Chaque produit est testé sur un groupe de 6 animaux. Dans chaque expérience sont inclus un groupe placebo (contrôle) et un groupe d'animaux traités avec un produit de référence (bézafibrate à la dose de 200 mg/kg/j). A la fin des traitements les animaux sont anesthésiés à l'éther. Le sang est prélevé par ponction abdominale et les triglycérides et le cholestérol total sont dosés dans les sérums. Cinq expériences indépendantes ont été réalisées selon ce protocole pour évaluer l'effet hypolipémiant de treize composés de cette invention.

### B. RESULTATS

Les résultats sont présentés dans le Tableau B. L'effet des traitements est exprimé en pourcentage de variation par rapport au groupe contrôle, calculé à partir des moyennes des valeurs absolues de triglycérides et de cholestérol total. Une valeur négative reflète donc un effet hypolipémiant du traitement indiqué. Les valeurs présentées pour le bézafibrate représentent les valeurs moyennes (± e.s.m) de cinq expériences.

**Tableau B**

| **Effet des produits sur les lipides plasmatiques** | | |
|---|---|---|
| **Composés** | **Triglycérides (% de variation/contrôle)** | **Cholestérol Total (% de variation/contrôle)** |
| Exemple 2 | -82 | -52 |
| Exemple 3 | -87 | -77 |
| Exemple 4 | -77 | -48 |
| Exemple 6 | -59 | -39 |
| Exemple 7 | -83 | -47 |
| Exemple 8 | -59 | -59 |
| Exemple 11 | -60 | -55 |
| Exemple 12 | -84 | -63 |
| Exemple 13 | -84 | -68 |
| Exemple 15 | -93 | -77 |
| Exemple 16 | -54 | -66 |
| Exemple 18 | -79 | -61 |
| Exemple 20 | -71 | -58 |
| Bézafibrate | -46 ± 19 | -48 ± 4 |

Tous les composés testés diminuent les triglycérides plasmatiques. Les exemples 2, 3, 7, 12, 13 et 15 sont statistiquement au moins aussi efficaces que le bézafibrate. Ces 6 composés exercent aussi un effet hypocholestérolémiant au moins aussi important que le bézafibrate.

### 3. CONCLUSION

Les résultats présentés ci-dessus montrent que les composés de la présente invention associent une double activité:
- un effet protecteur contre l'oxydation des LDL, avec une efficacité 10 à 70 fois supérieure à celle des composés de référence, probucol et trolox.
- un effet hypocholestérolémiant et hypotriglycéridémiant. Parmi les produits testés, six possèdent une activité au moins aussi importante que celle du fibrate de référence, le bézafibrate.

Aucun produit de référence n'associe ces deux effets.

## Revendications

1. Les acides et esters 2,2-diméthyl-ω-phénoxy alcanoïques substitués de formule I: 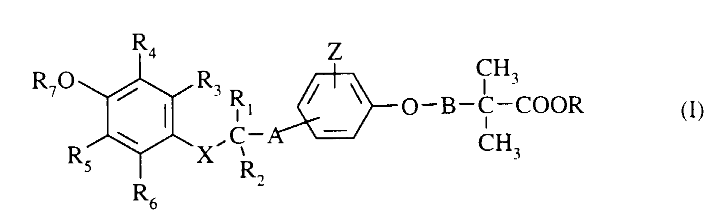 dans laquelle :
**X** représente un atome d'oxygène, un atome de soufre ou une liaison simple ;
**A** représente une liaison simple ou un radical hydrocarboné contenant de 1 à 9 atomes de carbone en chaîne droite ou ramifiée renfermant éventuellement une double liaison ou un atome d'oxygène ;
**B** représente un radical hydrocarboné contenant de 1 à 9 atomes de carbone en chaîne droite ou ramifiée ;
**R** représente un atome d'hydrogène ou un radical alkyle de 1 à 6 atomes de carbone en chaîne droite ou ramifiée, éventuellement substitué par un ou deux radicaux hydroxy ;
**R**_{**1**} **et R**_{**3**} représentent:
- chacun simultanément un atome d'hydrogène, ou
- forment ensemble un pont (CH₂)ₙ dans lequel n prend les valeurs 1 ou 2 sauf dans le cas où X rerpésente une liaison simple, ou
- R₁ représente
- un radical méthyle, ou
- une liaison simple formant une double liaison avec le groupe A lorsque celui--ci est un radical hydrocarboné et,
. dans chacun de ces cas, simultanément R₃ représente un atome d'hydrogène ;
**R**_{**2**} **et R**_{**6**} identiques ou différents, représentent chacun un atome d'hydrogène ou un radical méthyle ;
**R**_{**4**} **et R**_{**5**} identiques ou différents, représentent chacun un radical alkyle de 1 à 6 atomes de carbone en chaîne droite ou ramifiée ;
**R**_{**7**} représente un atome d'hydrogène ou un groupement protecteur labile tel que par exemple, un radical CH₃CO-, C₂H₅OCH₂- ou benzyle ; et
**Z** représente un atome d'hydrogène ou d'halogène ou un radical alkyle ou alkoxy contenant chacun de 1 à 5 atomes de carbone en chaîne droite ou ramifiée ;
et quand ils existent, les énantiomères et diastéréoisomères correspondants, ainsi que les sels physiologiquement tolérables des composés de formule I avec des bases appropriées.

2. Un composé de la revendication 1 qui est l'acide 2,2-diméthyl-5-{4-[2-(3,5-ditert-butyl-4-hydroxyphénylthio)éthyl]phénoxy} pentanoïque et son sel de tert-butylamine.

3. Un composé de la revendication 1 qui est l'acide 2,2-diméthyl-5-{4-[3-(3,5-ditert-butyl-4-hydroxyphénylthio)propyl]phénoxy} pentanoïque et son sel de tert-butylamine.

4. Un composé de la revendication 1 qui est l'acide 2,2-diméthyl-5-{3-[2-(4-hydroxy-2,3,5-triméthylphénylthio)éthyl]phénoxy} pentanoïque.

5. Un composé de la revendication 1 qui est l'acide 2,2-diméthyl-5-{4-[3-(3,5-ditert-butyl-4-hydroxyphénoxy)propyloxy]phénoxy} pentanoïque et son sel de tert-butylamine.

6. Un composé de la revendication 1 qui est l'acide 2,2-diméthyl-5-{3-[2-(3,5-ditert-butyl-4-hydroxyphénoxy)éthyl]phénoxy} pentanoïque et son sel de tert-butylamine.

7. Un composé de la revendication 1 qui est l'acide 2,2-diméthyl-5-{4-[3-(4-hydroxy-2,3,5-triméthylphénoxy)propyl]phénoxy}pentanoïque et son sel de tert-butylamine.

8. Le procédé de préparation des composés de la revendication 1 caractérisé en ce que l'on fait réagir :
a)
. soit un composé de formule IIa : 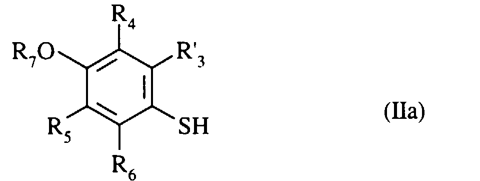 dans laquelle :
R₄, R₅, R₆ et R₇ ont les significations définies dans la revendication 1 et
R'₃ représente un atome d'hydrogène,
. avec un composé de formule IIIa: 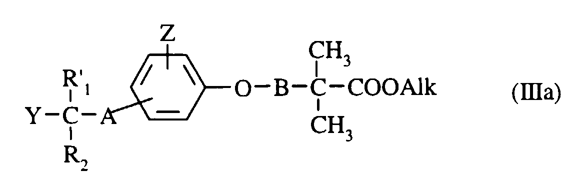 dans laquelle :
A, B, R₂ et Z ont les significations définies dans la revendication 1,
R'₁ représente un atome d'hydrogène ou un radical méthyle ;
Alk représente un radical alkyle de 1 à 6 atomes de carbone en chaîne droite ou ramifiée, et
Y représente un atome de chlore ou de brome ;
. pour obtenir un composé de formule Ia₁ : 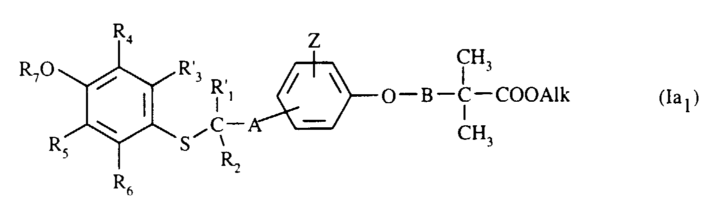 dans laquelle R'₁, R₂, R'₃, R₄, R₅, R₆, R₇, A, Z, B et Alk ont les significations précédemment définies,
. lequel composé Ia₁ est, selon la nature de R₇, transformé par des méthodes séquentielles de saponification, hydrolyse ou hydrogénolyse, en un composé de formule Ia₂ : 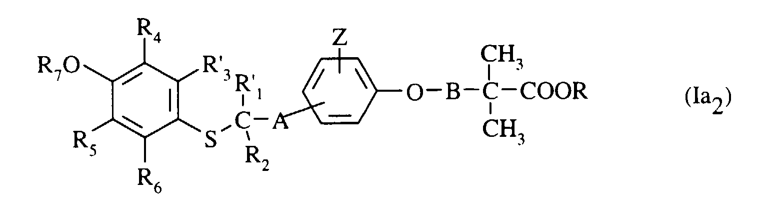 dans laquelle R'₁, R₂, R'₃, R₄, R₅, R₆, R₇, A, Z et B ont les significations précédemment définies et R a la signification définie dans la revendication 1 ;
**b)**
. soit un composé de formule IIb : 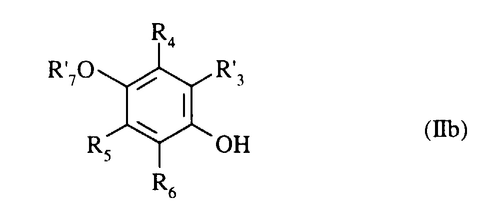 dans laquelle :
R'₃, R₄, R₅ et R₆ ont les significations précédemment définies, et
R'₇ représente un groupement protecteur labile ;
. avec un composé de formule IIIb : 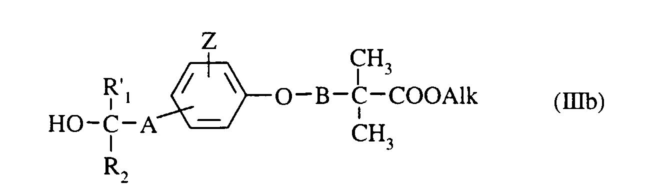 dans laquelle R'₁, R₂, A, Z, B et Alk ont les significations précédemment définies,
. pour obtenir un composé de formule Ib₁ : 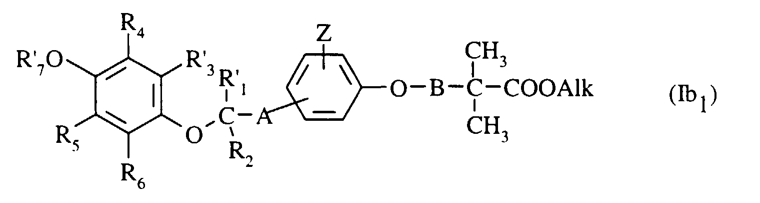 dans laquelle R'₁, R₂, R'₃, R₄, R₅, R₆, R'₇, A, Z, B et Alk ont les significations précédemment définies,
. lequel composé de formule Ib₁ est, selon la nature de R'₇, transformé par des méthodes séquentielles de saponification, hydrolyse ou hydrogénolyse, en un composé de formule Ib₂: 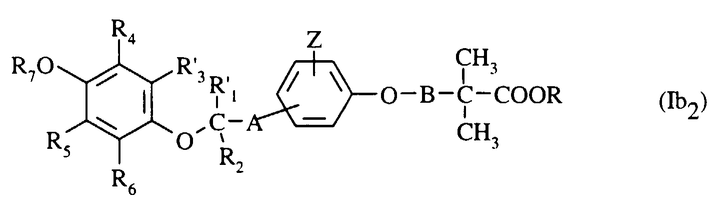 dans laquelle R'₁, R₂, R'₃, R₄, R₅, R₆, R₇, A, Z, B et R ont les significations précédemment définies ;
c)
. soit un composé de formule IIc : 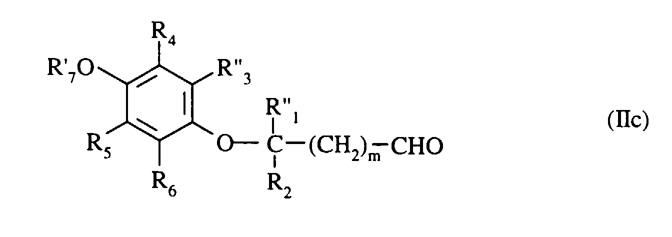 dans laquelle :
R₂, R₄, R₅, R₆ et R'₇ ont les significations précédemment définies,
R"₁ et R"₃ représentent ensemble un pont (CH₂)ₙ dans lequel n prend la signification définie dans la revendication 1, et
m représente zéro ou un nombre entier de 1 à 5 inclus ;
. avec un composé de formule IIIc : 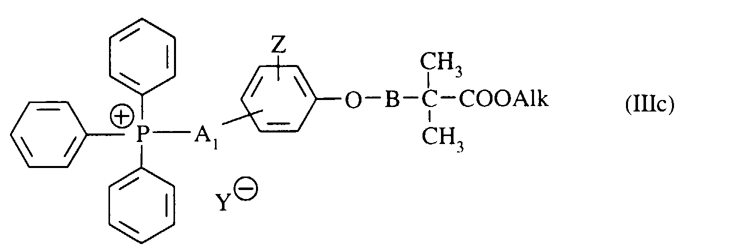 dans laquelle :
Alk, B, Z et Y ont les significations précédemment défines, et
A₁ représente un radical hydrocarboné contenant de 1 à 3 atomes de carbone en chaîne droite ou ramifiée ;
. pour obtenir un composé de formule Ic₁ : 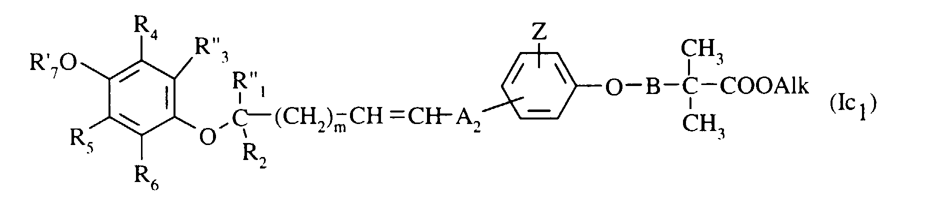 dans laquelle :
R"₁, R₂, R"₃, R₄, R₅, R₆, R'₇, m, Z, B et Alk ont les significations précédemment définies, et
A₂ représente une liaison simple ou un radical hydrocarboné contenant 1 ou 2 atomes de carbone en chaîne droite ou ramifiée ;
. lequel composé de formule Ic₁ est réduit pour obtenir le composé de formule Ic₂ : 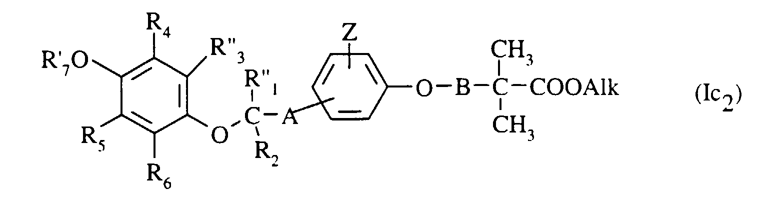 dans laquelle R"₁, R₂, R"₃, R₄, R₅, R₆, R'₇, A, Z, B et Alk ont les significations précédemment définies ;
. lequel composé de formule Ic₂ est, selon la nature de R'₇, transformé, par des méthodes séquentielles de saponification, hydrolyse ou hydrogénolyse en un composé de formule Ic₃ : 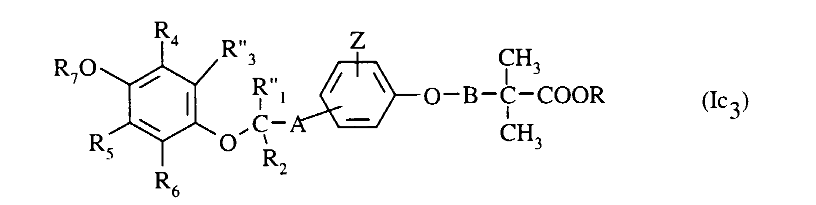 dans laquelle R"₁, R₂, R"₃, R₄, R₅, R₆, R₇, A, Z, B et R ont les significations précédemment définies.

9. Les compositions pharmaceutiques ayant une activité protectrice de l'oxydation des LDL humaines et une activité hypolipémiante, contenant comme principe actif un composé selon l'une quelconque des revendications 1 à 7 avec un ou plusieurs excipients pharmaceutiques appropriés.

10. Les compositions pharmaceutiques selon la revendication 9 présentées sous une forme convenant pour le traitement des hypercholestérolémies, des hypertriglycéridémies, des dyslipémies, du diabète, de l'athérosclérose et des pathologies dans lesquelles une peroxydation lipidique membranaire joue un rôle initiateur et/ou aggravant.

## Claims

1. Substituted 2,2-dimethyl-ω-phenoxyalkanoic acids and esters of formula I: 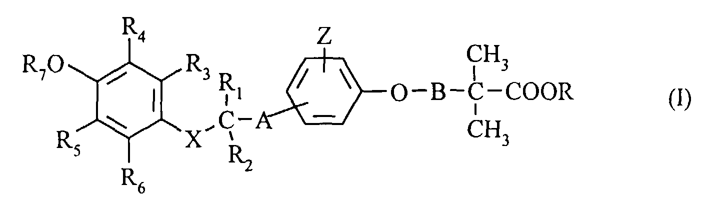 wherein :
**X** represents an oxygen atom, a sulphur atom or a single bond;
**A** represents a single bond, or a hydrocarbon radical having from 1 to 9 carbon atoms in straight or branched chain and optionally containing a double bond or an oxygen atom;
**B** represents a hydrocarbon radical having from 1 to 9 carbon atoms in straight or branched chain;
**R** represents a hydrogen atom, or an alkyl radical having from 1 to 6 carbon atoms in straight or branched chain that is optionally substituted by one or two hydroxy radicals;
**R**_{**1**} **and R**_{**3**} **:**
- each simultaneously represents a hydrogen atom, or
- together form a (CH₂)ₙ bridge wherein n has a value of 1 or 2 except in the case where X represents a single bond, or
- R₁ represents
- a methyl radical, or
- a single bond forming a double bond with the group A when that group is a hydrocarbon radical and,
. in each of those cases R₃ simultaneously represents a hydrogen atom;
**R**_{**2**} **and R**_{**6**}, which are identical or different, each represents a hydrogen atom or a methyl radical;
**R**_{**4**} **and R**_{**5**}, which are identical or different, each represents an alkyl radical having from 1 to 6 carbon atoms in straight or branched chain;
**R**_{**7**} represents a hydrogen atom or a labile protecting group such as, for example, a CH₃CO-, C₂H₅OCH₂- or benzyl radical; and
**Z** represents a hydrogen or halogen atom or an alkyl or alkoxy radical each having from 1 to 5 carbon atoms in straight or branched chain,
and, when they exist, the corresponding enantiomers and diastereoisomers, and also the physiologically tolerable salts of compounds of formula I with appropriate bases.

2. A compound of claim 1 which is 2,2-dimethyl-5-{4-[2-(3,5-di-tert-butyl-4-hydroxyphenylthio)ethyl]phenoxy}pentanoic acid and its tert-butylamine salt.

3. A compound of claim 1 which is 2,2-dimethyl-5-{4-[3-(3,5-di-tert-butyl-4-hydroxyphenylthio)propyl]phenoxy}pentanoic acid and its tert-butylamine salt.

4. A compound of claim 1 which is 2,2-dimethyl-5-{3-[2-(4-hydroxy-2,3,5-trimethylphenylthio)ethyl]phenoxy}pentanoic acid.

5. A compound of claim 1 which is 2,2-dimethyl-5-{4-[3-(3,5-di-tert-butyl-4-hydroxyphenoxy)propyloxy]phenoxy}pentanoic acid and its tert-butylamine salt.

6. A compound of claim 1 which is 2,2-dimethyl-5-{3-[2-(3,5-di-tert-butyl-4-hydroxyphenoxy)ethyl]phenoxy}pentanoic acid and its tert-butylamine salt.

7. A compound of claim 1 which is 2,2-dimethyl-5-{4-[3-(4-hydroxy-2,3,5-trimethylphenoxy)propyl]phenoxy}pentanoic acid and its tert-butylamine salt.

8. A process for the preparation of compounds of claim 1, characterised in that:
**a)**
. a compound of formula lla : 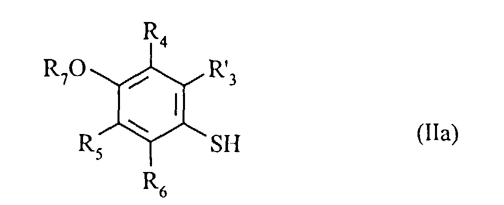 wherein :
R₄, R₅, R₆ and R₇ are as defined in claim 1 and
R'₃ represents a hydrogen atom,
. is reacted with a compound of formula llla : 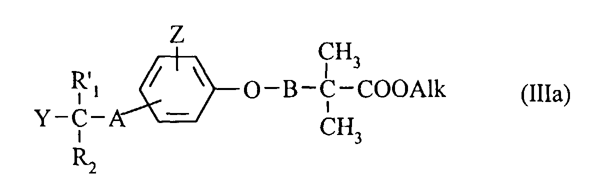 wherein :
A, B, R₂ and Z are as defined in claim 1,
R'₁ represents a hydrogen atom or a methyl radical ;
Alk represents an alkyl radical having from 1 to 6 carbon atoms in straight or branched chain, and
Y represents a chlorine or bromine atom ;
. to obtain a compound of formula la₁ : 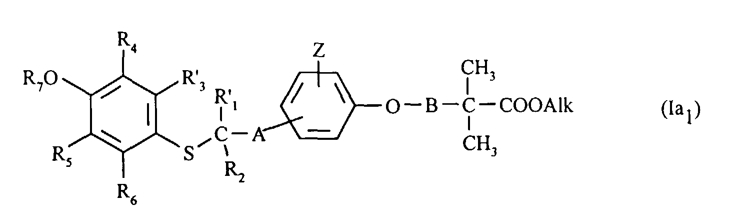 wherein R'₁, R₂, R'₃, R₄, R₅, R₆, R₇, A, Z, B and Alk are as defined above,
. which compound la₁ is, depending on the nature of R₇, converted by sequential methods of saponification, hydrolysis or hydrogenolysis into a compound of formula la₂ : 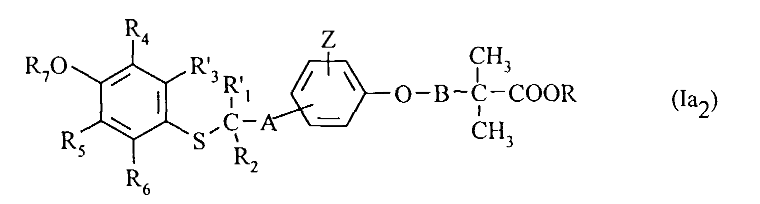 wherein R'₁, R₂, R'₃, R₄, R₅, R₆, R₇, A, Z and B are as defined above and R is as defined in claim 1;
**b)**
. or a compound of formula llb : 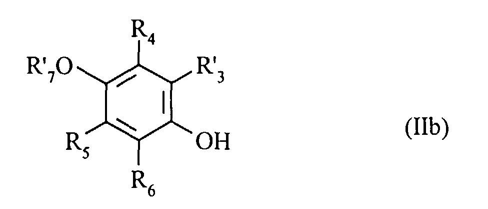 wherein :
R'₃, R₄, R₅ and R₆ are as defined above, and
R'₇ represents a labile protecting group;
. is reacted with a compound of formula lllb : 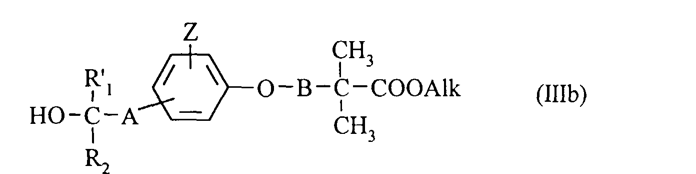 wherein R'₁, R₂, A, Z, B and Alk are as defined above,
. to obtain a compound of formula lb₁ : 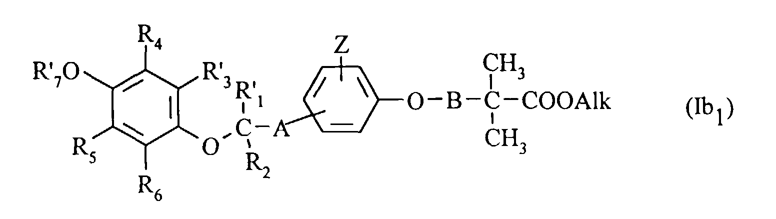 wherein R'₁, R₂, R'₃, R₄, R₅, R₆, R'₇, A, Z, B and Alk are as defined above,
. which compound of formula lb₁ is, depending on the nature of R'₇, converted by sequential methods of saponification, hydrolysis or hydrogenolysis into a compound of formula lb₂ : 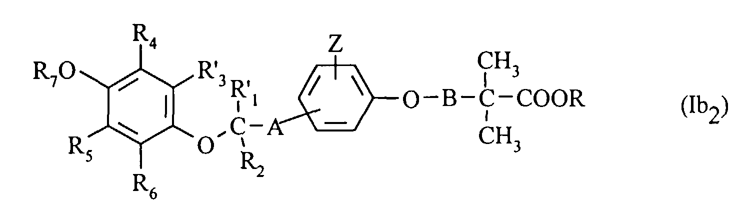 wherein R'₁, R₂, R'₃, R₄, R₅, R₆, R₇, A, Z, B and R are as defined above ;
c)
. or a compound of formula llc : 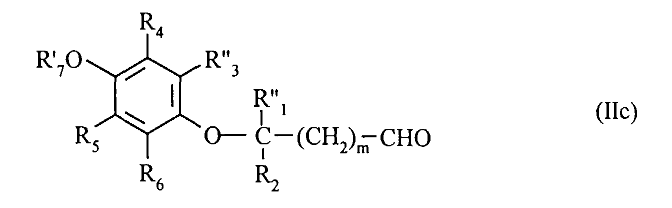 wherein :
R₂, R₄, R₅, R₆ and R'₇ are as defined above,
R"₁ and R"₃ together represent a (CH₂)ₙ bridge wherein n is as defined in claim 1, and
m represents zero or an integer of from 1 to 5 inclusive ;
. is reacted with a compound of formula lllc : 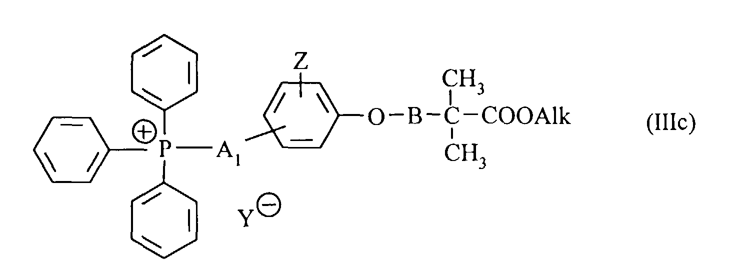 wherein :
Alk, B, Z and Y are as defined above and
A₁ represents a hydrocarbon radical containing from 1 to 3 carbon atoms in straight or branched chain ;
. to obtain a compound of formula lc₁ : 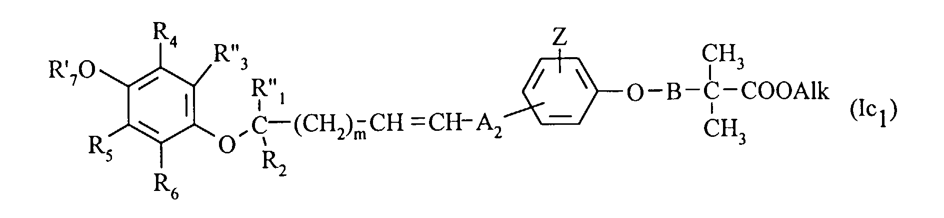 wherein :
R"₁, R₂, R"₃, R₄, R₅, R₆, R'₇, m, Z, B and Alk are as defined above and
A₂ represents a single bond or a hydrocarbon radical containing 1 or 2 carbon atoms in straight or branched chain;
. which compound of formula Ic₁ is reduced to obtain the compound of formula lc₂ : 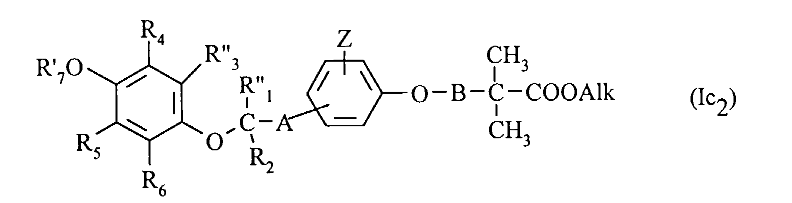 wherein R"₁, R₂, R"₃, R₄, R₅, R₆, R'₇, A, Z, B and Alk are as defined above ;
. which compound of formula Ic₂ is, depending on the nature of R'₇, converted by sequential methods of saponification, hydrolysis or hydrogenolysis into a compound of formula lc₃ : 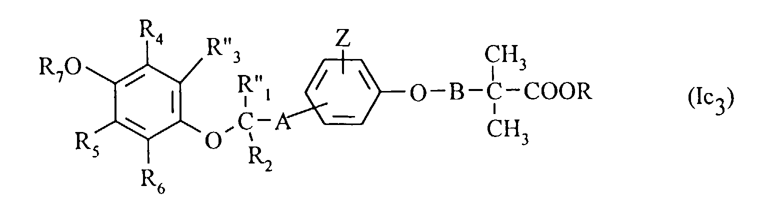 wherein R"₁, R₂, R"₃, R₄, R₅, R₆, R₇, A, Z, B and R are as defined above.

9. Pharmaceutical compositions having a protective activity against the oxidation of human LDLs and a hypolipaemic activity, comprising as active ingredient a compound according to any one of claims 1 to 7 with one or more appropriate pharmaceutical excipients.

10. Pharmaceutical compositions according to claim 9 presented in a form suitable for the treatment of hypercholesterolaemia, hypertriglyceridaemia, dyslipaemia, diabetes, atherosclerosis and pathologies in which membrane lipid peroxidation plays an initiating and/or aggravating role.

## Patentansprüche

1. Substituierte 2,2-Dimethyl-ω-phenoxy-alkansäuren und deren Ester der Formel I: 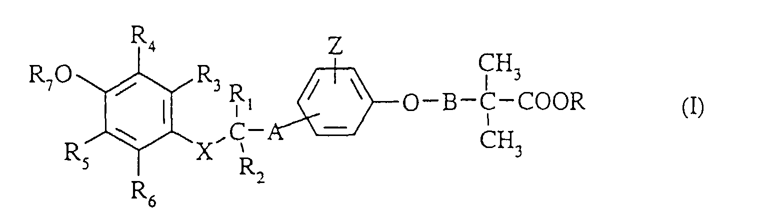 in der:
**X** ein Sauerstoffatom, ein Schwefelatom oder eine Einfachbindung bedeutet;
**A** eine Einfachbindung oder eine Kohlenwasserstoffgruppe, die 1 bis 9 Kohlenstoffatome in gerader oder verzweigter Kette aufweist und gegebenenfalls eine Doppelbindung oder ein Sauerstoffatom enthält;
**B** eine Kohlenwasserstoffgruppe bedeutet, die 1 bis 9 Kohlenstoffatome in gerader oder verzweigter Kette enthält;
**R** ein Wasserstoffatom oder eine Alkylgruppe mit 1 bis 6 Kohlenstoffatomen in gerader oder verzweiger Kette, die gegebenenfalls durch eine oder zwei Hydroxygruppen substituiert ist, bedeutet;
**R**_{**1**} **und R**_{**3**} **:**
- jeweils gleichzeitig ein Wasserstoffatom bedeuten, oder
- gemeinsam eine Brücke (CH₂)ₙ bilden, worin n die Werte 1 oder 2 besitzt mit Ausnahme des Falls, da X eine Einfachbindung darstellt, oder
- R₁
- eine Methylgruppe oder
- eine Einfachbindung darstellt, die mit der Gruppe A, wenn diese einen Kohlenwasserstoffrest bedeutet, eine Doppelbindung bildet und
- in jedem dieser Fälle R₃ gleichzeitig ein Wasserstoffatom darstellt;
**R**_{**2**} **und R**_{**6**}, die gleichartig oder verschieden sind, jeweils ein Wasserstoffatom oder eine Methylgruppe bedeuten;
**R**_{**4**} **und R**_{**5**}, die gleichartig oder verschieden sind, jeweils eine Alkylgruppe mit 1 bis 6 Kohlenstoffatomen in gerader oder verzweigter Kette bedeuten;
**R**_{**7**} ein Wasserstoffatom oder eine labile Schutzgruppe, wie beispielsweise eine Gruppe CH₃CO-, C₂H₅OCH₂- oder eine Benzylgruppe bedeutet; und
**Z** ein Wasserstoffatom oder ein Halogenatom oder eine Alkyl- oder Alkoxygruppe, die jeweils 1 bis 5 Kohlenstoffatome in gerader oder verzweigter Kette aufweisen, bedeutet;
und, falls sie existieren, die entsprechenden Enantiomere und Diastereoisomere sowie die physiologisch verträglichen Salze der Verbindungen der Formel I mit geeigneten Basen.

2. Verbindung nach Anspruch 1, nämlich 2,2-Dimethyl-5-{4-[2-(3,5-ditert.-butyl-4-hydroxyphenylthio)-ethyl]-phenoxy}-pentansäure und deren tert.-Butylaminsalz.

3. Verbindung nach Anspruch 1, nämlich 2,2-Dimethyl-5-{4-[3-(3,5-ditert.-butyl-4-hydroxyphenylthio)-propyl]-phenoxy}-pentansäure und deren tert.-Butylaminsalz.

4. Verbindung nach Anspruch 1, nämlich 2,2-Dimethyl-5-{3-[2-(4-hydroxy-2,3,5-trimethylphenylthio)-ethyl]-phenoxy}-pentansäure.

5. Verbindung nach Anspruch 1, nämlich 2,2-Dimethyl-5-{4-[3-(3,5-ditert.-butyl-4-hydroxyphenoxy)-propyloxy]-phenoxy}-pentansäure und deren tert.-Butylaminsalz.

6. Verbindung nach Anspruch 1, nämlich 2,2-Dimethyl-5-{3-[2-(3,5-ditert.-butyl-4-hydroxyphenoxy)-ethyl]-phenoxy}-pentansäure und deren tert.-Butylaminsalz.

7. Verbindung nach Anspruch 1, nämlich 2,2-Dimethyl-5-{4-[3-(4-hydroxy-2,3,5-trimethylphenoxy)-propyl]-phenoxy}-pentansäure und deren tert.-Butylaminsalz.

8. Verfahren zur Herstellung der Verbindungen nach Anspruch 1, dadurch gekennzeichnet, daß man:
**a)**
. entweder eine Verbindung der Formel IIa: 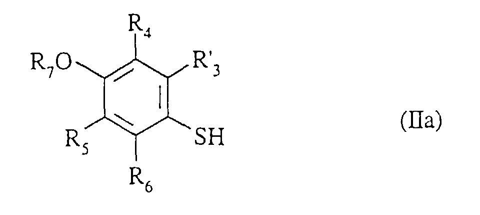 in der:
R₄, R₅, R₆ und R₇ die in Anspruch 1 angegebenen Bedeutungen besitzen und
R'₃ ein Wasserstoffatom darstellt,
. mit einer Verbindung der Formel IIIa umsetzt: 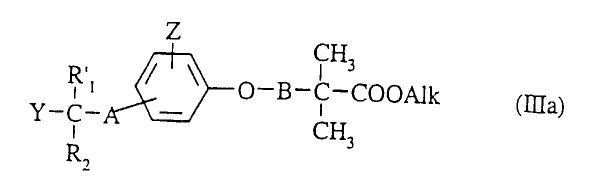 in der:
A, B, R₂ und Z die in Anspruch 1 angegebenen Bedeutungen besitzen,
R'₁ ein Wasserstoffatom oder eine Methylgruppe,
Alk eine Alkylgruppe mit 1 bis 6 Kohlenstoffatomen in gerader oder verzweigter Kette und
Y ein Chlor- oder Bromatom bedeuten,
. zur Bildung einer Verbindung der Formel Ia₁: 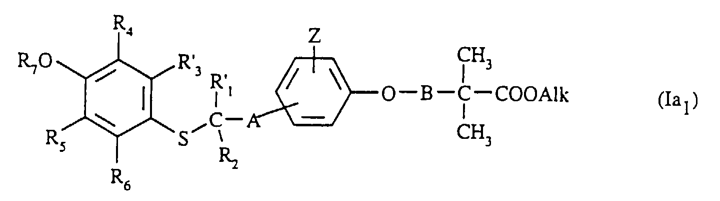 in der R'₁, R₂, R'₃, R₄, R₅, R₆, R₇, A, Z, B und Alk die oben angegebenen Bedeutungen besitzen,
. welche Verbindung Ia₁ in Abhängigkeit von der Art von R₇ mit Hilfe nacheinander folgender Methoden der Verseifung, Hydrolyse oder Hydrogenolyse in eine Verbindung der Formel Ia₂ umgewandelt wird: 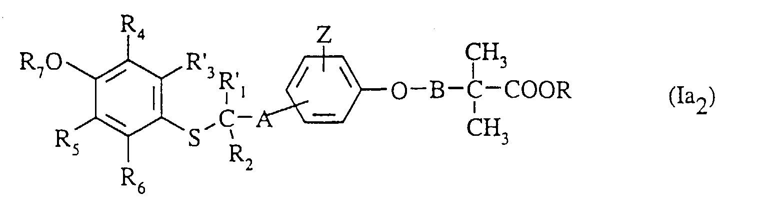 in der R'₁, R₂, R'₃, R₄, R₅, R₆, R₇, A, Z und B die oben angegebenen Bedeutungen besitzen und R die in Anspruch 1 angegebene Bedeutung besitzt;
**b)**
. oder eine Verbindung der Formel IIb: 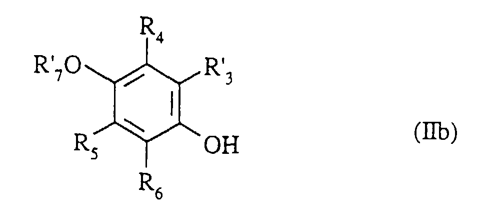 in der:
R'₃, R₄, R₅ und R₆ die oben angegebenen Bedeutungen besitzen und
R'₇ eine labile Schutzgruppe bedeutet;
. mit einer Verbindung der Formel IIIb umsetzt: 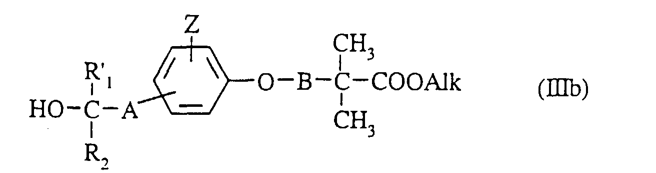 in der R'₁, R₂, A, Z, B und Alk die oben angegebenen Bedeutungen besitzen,
. zur Bildung einer Verbindung der Formel Ib₁: 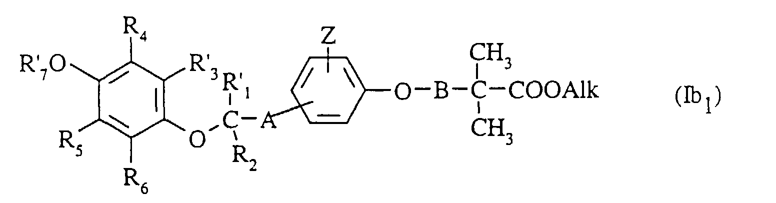 in der R'₁, R₂, R'₃, R₄, R₅, R₆, R'₇, A, Z, B und Alk die oben angegebenen Bedeutungen besitzen,
. welche Verbindung der Formel Ib₁ in Abhängigkeit von der Art von R'₇ mit Hilfe aufeinanderfolgender Methoden der Verseifung, Hydrolyse oder Hydrogenolyse in eine Verbindung der Formel Ib₂ umgewandelt wird: 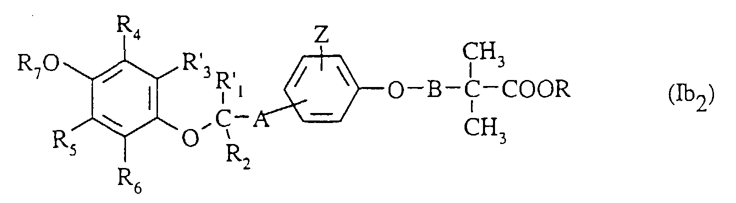 in der R'₁, R₂, R'₃, R₄, R₅, R₆, R₇, A, Z, B und R die oben angegebenen Bedeutungen besitzen;
**c)**
. oder eine Verbindung der Formel IIc: 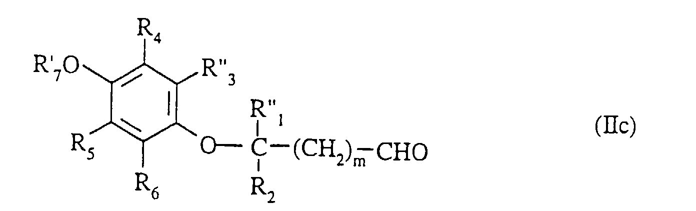 in der:
R₂, R₄, R₅, R₆ und R'₇ die oben angegebenen Bedeutungen besitzen,
R"₁ und R"₃ gemeinsam eine Brücke (CH₂)ₙ bilden, worin n die in Anspruch 1 angegebenen Bedeutungen besitzt, und
m Null oder eine ganze Zahl von 1 bis 5 einschließlich bedeutet;
. mit einer Verbindung der Formel IIIc umsetzt: 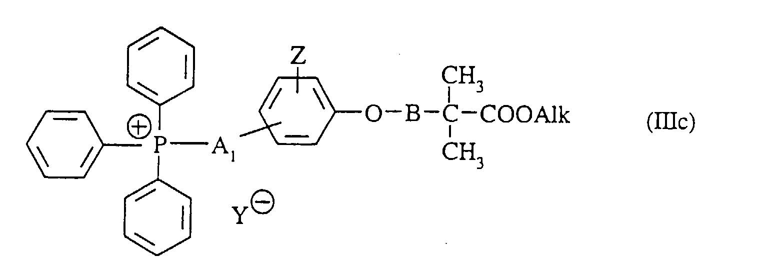 in der:
Alk, B, Z und Y die oben angegebenen Bedeutungen besitzen und
A₁ eine Kohlenwasserstoffgruppe mit 1 bis 3 Kohlenstoffatomen in gerader oder verzweigter Kette darstellt;
. zur Bildung einer Verbindung der Formel Ic₁: 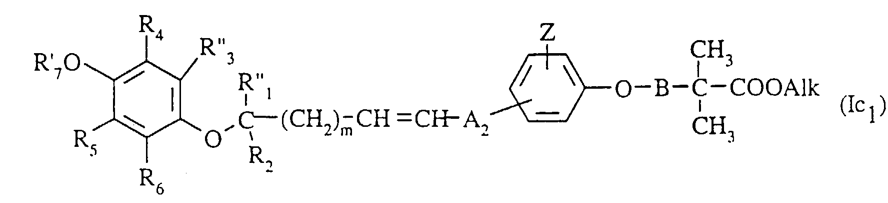 in der:
R"₁, R₂, R"₃, R₄, R₅, R₆, R'₇, m, Z, B und Alk die oben an gegebenen Bedeutungen besitzen und
A₂ eine Einfachbindung oder eine Kohlenwasserstoffgruppe, die ein oder zwei Kohlenstoffatome in gerader oder verzweigter Kette enthält, bedeutet;
. welche Verbindung der Formel lc₁ reduziert wird zur Bildung der Verbindung der Formel Ic₂: 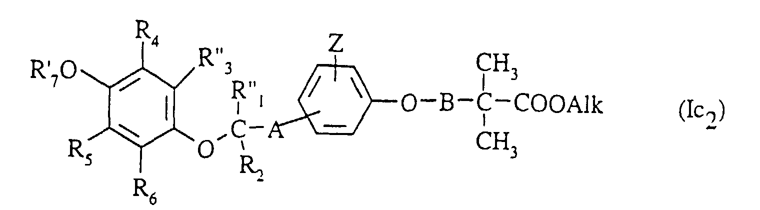 in der R"₁, R₂, R"₃, R₄, R₅, R₆, R'₇, A, Z, B und Alk die oben angegebenen Bedeutungen besitzen;
. welche Verbindung der Formel Ic₂ in Abhängigkeit von der Art von R'₇ mit Hilfe aufeinanderfolgender Verfahren der Verseifung, der Hydrolyse oder der Hydrogenolyse in eine Verbindung der Formel Ic₃ umgewandelt wird: 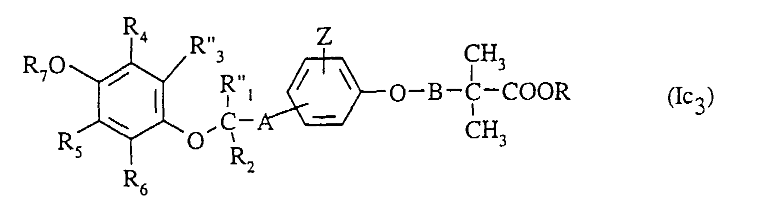 in der R"₁, R₂, R"₃, R₄, R₅, R₆, R₇, A, Z, B und R die oben angegebenen Bedeutungen besitzen.

9. Pharmazeutische Zubereitungen mit einer Schutzwirkung gegen die Oxidation von menschlichen LDL und mit einer hypolipämischen Wirkung, enthaltend als Wirkstoff eine Verbindung nach einem der Ansprüche 1 bis 7 zusammen mit einem oder mehreren geeigneten pharmazeutischen Trägermaterialien.

10. Pharmazeutische Zubereitungen nach Anspruch 9 in einer Form, die zur Behandlung von Hypercholesterinämien, Hypertriglyceridämien, Dyslipämien, Diabetes, Atherosklerose und pathologischen Zuständen, bei denen eine Membranlipid-Peroxidation eine auslösende und/oder erschwerende Rolle spielt, geeignet sind.
